# EUROPÄISCHE PATENTSCHRIFT

(11) **EP 0 914 087 B1**
(45) Veröffentlichungstag und Bekanntmachung des Hinweises auf die Patenterteilung: **06.02.2002**
(21) Anmeldenummer: 97927110.3
(22) Anmeldetag: 03.06.1997
(51) Int. Cl.: A61K 7/48

(54) **VERWENDUNG VON FETTSTOFFEN ALS SILICONERSATZ ZUR HERSTELLUNG VON KOSMETISCHEN UND/ODER PHARMAZEUTISCHEN ZUBEREITUNGEN**
USE OF FATS TO REPLACE SILICONE IN THE PRODUCTION OF COSMETIC AND/OR PHARMACEUTICAL PREPARATIONS
UTILISATION DE SUBSTANCES GRASSES COMME SUBSTITUTS DE SILICONE POUR PRODUIRE DES PREPARATIONS COSMETIQUES ET/OU PHARMACEUTIQUES

(30) Priorität: 12.06.1996 DE 19623383
(43) Veröffentlichungstag der Anmeldung: 12.05.1999
(73) Patentinhaber: Cognis Deutschland GmbH, 40589 Düsseldorf (DE)
(72) Erfinder: KAHRE, Jörg, D-40789 Monheim (DE); ANSMANN, Achim, D-40699 Erkrath (DE); FABRY, Bernd, D-41352 Korschenbroich (DE); KAWA, Rolf, D-40789 Monheim (DE); SEIPEL, Werner, D-40723 Hilden (DE)
(86) Internationale Anmeldenummer: EP9702866
(87) Internationale Veröffentlichungsnummer: WO9747281

(56) Entgegenhaltungen:
- EP-A- 0 603 078
- DE-A- 4 129 986
- DE-A- 4 420 880

## Beschreibung

### Gebiet der Erfindung

Die Erfindung betrifft die Verwendung von ausgewählten Fettstoffen als Ersatz für Silicone bei der Herstellung von kosmetischen und/oder pharmazeutischen Zubereitungen.

### Stand der Technik

Silicone werden in der Haut- und Haarkosmetik als Additive zur Beeinflussung von Griff und Glanz eingesetzt. Nachteilig hierbei ist jedoch der sogenannte "build-up"-Effekt. Hierunter ist zu verstehen, daß bei wiederholter Anwendung siliconhaltiger Produkte sich auf der Haut oder dem Haar eine Schicht von Polymeren aufbaut, welche durch einfaches Waschen nur schwer zu entfernen ist. Insbesondere beim Haar kann es dabei zu einer Belastung und einer möglichen Beeinträchtigung weiterer Behandlungen wie z.B. beim Wellen oder Färben kommen. Eine Übersicht zum Einsatz von Siliconen in der Kosmetik findet sich beispielsweise von K.Schnurrbusch in **Seifen-Fette-Öle-Wachse 100, 173, 1974).**

Die Aufgabe der Erfindung hat somit darin bestanden, Ersatzstoffe für Silicone zu finden, die sich bei der Anwendung nicht anreichern und dennoch hinsichtlich Griff und Glanz mindestens vergleichbare anwendungstechnische Eigenschaften aufweisen.

In DE-A1-4129986 werden Ester öle, in DE-A1-4119890 Guerbet carbonate als Ersatz für Silikon öle beschrieben.

### Beschreibung der Erfindung

Gegenstand der Erfindung ist die Verwendung von Fettstoffen als Siliconersatz zur Herstellung von kosmetischen und/oder pharmazeutischen Zubereitungen, die sich dadurch auszeichnet, daß man Ölkörper einsetzt, die ausgewählt sind aus der Gruppe, die gebildet wird von
(a) Dialkylethern,
(b) Dialkylcyclohexanen,
(c) Guerbetalkoholen,
(d) Polyolpolyhydroxystearaten und/oder
(e) Hydroxycarbonsäureestern.

Überraschenderweise wurde gefunden, daß die ausgewählten Ölkörper im Vergleich zu Siliconen hinsichtlich Griff und Glanz mindestens vergleichbar gut sensorisch beurteilt werden, ohne daß es auf Haut und Haaren zu einer unerwünschten Anreicherung kommt.

### Dialkylether

Dialkylether, die die Komponente (a) bilden, folgen der Formel (I),

**R**^{**1**}**-O-R**^{**2**} **(I)**

in der R¹ und R² unabhängig voneinander für einen linearen oder verzweigten Alkyl- und/oder Alkenylrest mit 6 bis 22, vorzugsweise 8 bis 18 und insbesondere 12 bis 18 Kohlenstoffatomen steht. Die Ether können asymmetrisch, vorzugsweise aber symmetrisch aufgebaut sein. Typische Beispiele sind Di-n-octylether, Di-i-octylether und Di-n-stearylether.

### Dialkylcyclohexane

Dialkylcyclohexane, die die Komponente (b) bilden, folgen der Formel (II),

**R**^{**3**}**-[C]-R**^{**4**} **(II)**

in der R³ und R⁴ unabhängig voneinander für einen linearen oder verzweigten Alkyl- und/oder Alkenylrest mit 6 bis 22, vorzugsweise 8 bis 18 und insbesondere 12 bis 18 Kohlenstoffatomen und C für einen Cyclohexylrest steht. Typische Beispiele sind Di-n-octylcyclohexan, Di-i-octylcyclohexan und Di-n-stearylcyclohexan.

### Guerbetalkohole

Guerbetalkohole, die die Komponente (c) bilden, werden vorzugsweise durch basenkatalysierte Eigenkondensation von linearen und/oder verzweigten Alkoholen mit 6 bis 22 und vorzugsweise 8 bis 18 Kohlenstoffatomen erhalten. Eine Übersicht hierzu findet sich von A.J.O'Lennick in **Soap Cosm.Chem.Spec. (April) 52 (1987).** Typische Beispiele sind Kondensationsprodukte von technischen Fettalkoholschnitten mit 8 bis 10 bzw. 16 bis 18 Kohlenstoffatomen.

### Polyolpolyhydroxystearate

Die Polyolkomponente der Polyolpolyhydroxystearate, die die Komponente (f) bilden, kann sich von Stoffen ableiten, die über mindestens zwei, vorzugsweise 3 bis 12 und insbesondere 3 bis 8 Hydroxylgruppen und 2 bis 12 Kohlenstoffatome verfügen. Typische Beispiele sind:
- Glycerin und Polyglycerin;
- Alkylenglycole, wie beispielsweise Ethylenglycol, Diethylenglycol, Propylenglycol;
- Methyolverbindungen, wie insbesondere Trimethylolethan, Trimethylolpropan, Trimethylolbutan, Pentaerythrit und Dipentaerythrit;
- Alkyloligoglucoside mit 1 bis 22, vorzugsweise 1 bis 8 und insbesondere 1 bis 4 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker wie beispielsweise Glucamin.

Unter den Stoffen der Komponente (f) kommt Umsetzungsprodukten auf Basis von Polyglycerin wegen ihrer ausgezeichneten anwendungstechnischen Eigenschaften eine besondere Bedeutung zu. Als besonders vorteilhaft hat sich die Verwendung von ausgewählten Polyglycerinen erwiesen, die die folgende Homologenverteilung aufweisen (in Klammern angegeben sind die bevorzugten Bereiche):

| | |
|---|---|
| Glycerin | 5 bis 35 (15 bis 30) Gew.-% |
| Diglycerine | 15 bis 40 (20 bis 32) Gew.-% |
| Triglycerine | 10 bis 35 (15 bis 25) Gew.-% |
| Tetraglycerine | 5 bis 20 (8 bis 15) Gew.-% |
| Pentaglycerine | 2 bis 10 (3 bis 8) Gew.-% |
| Oligoglycerine | ad 100 Gew.-% |

### Hydroxycarbonsäureester

Als letzte Komponente (g) kommen Ester von Hydroxycarbonsäuren mit 3 bis 18, vorzugsweise 3 bis 12 Kohlenstoffatomen mit aliphatischen Alkoholen mit 1 bis 22, vorzugsweise 6 bis 18 und insbesondere 12 bis 18 Kohlenstoffatomen in Betracht. Typische Beispiele sind Ester der Milchsäure, Äpfelsäure, Weinsäure, Citronensäure, Ricinolsäure und/oder 12-Hydroxystearinsäure mit Methanol, Ethanol, Capronalkohol, Caprylalkohol, 2-Ethylhexylalkohol, Caprinalkohol, Laurylalkohol, Isotridecylalkohol, Myristylalkohol, Ce-tylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen. Bevorzugt ist der Einsatz von langkettigen Hydroxycarbonsäuren, wie Ricinol- und Hydroxystearinsäure mit kurzkettigen Alkoholen, wie beispielsweise Methanol oder Ethanol oder kurzkettigen Hydroxycarbonsäuren, wie Milchsäure oder Citronensäurfe mit langkettigen Fettalkoholen wie beispielsweise Kokosfettalkohol oder Cetearylalkohol.

### Tenside

In einer bevorzugten Ausführungsform der Erfindung können die sensorischen Eigenschaften der Fettstoffe durch Abmischung mit nichtionischen Tensiden, vorzugsweise vom Typ der Alkyl- und/oder Alkenyloligoglykoside und/oder Fettsäure-N-alkylglucamide weiter verbessert werden. Dabei ist es möglich, die Fettstoffe und die nichtionischen Tenside im Gewichtsverhältnis 10 : 90 bis 90 : 10, vorzugsweise 25 : 75 bis 75 : 25 und insbesondere 40 : 60 bis 60 : 40 einzusetzen.

### Alkyl- und/oder Alkenyloligoglykoside

Alkyl- und Alkenyloligoglykoside stellen bekannte nichtionische Tenside dar, die der Formel **(IV)** folgen,

**R**^{**7**}**O-[G]**_{**p**} **(IV)**

in der R⁷ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Stellvertretend für das umfangreiche Schrifttum sei hier auf die Schriften **EP-A1 0 301 298** und **WO 90/03977** verwiesen.

Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkyl- und/oder Alkenyloligo**glucoside.** Die Indexzahl p in der allgemeinen Formel **(IV)** gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt.

Der Alkyl- bzw. Alkenylrest R⁷ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R⁷ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.

### Fettsäure-N-alkylpolyhydroxyalkylamide

Fettsäure-N-alkylpolyhydroxyalkylamide stellen nichtionische Tenside dar, die der Formel **(V)** folgen, 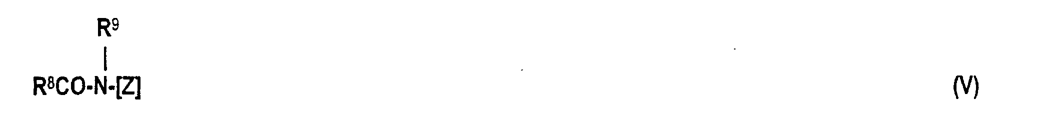 in der R⁸CO für einen aliphatischen Acylrest mit 6 bis 22 Kohlenstoffatomen, R⁹ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht. Bei den Fettsäure-N-alkylpolyhydroxyalkylamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können. Hinsichtlich der Verfahren zu ihrer Herstellung sei auf die US-Patentschriften **US 1,985,424, US 2,016,962** und **US 2,703,798** sowie die internationale Patentanmeldung **WO 92/06984** verwiesen. Eine Übersicht zu diesem Thema von H.Kelkenberg findet sich in **Tens.Surf. Deterg. 25, 8 (1988).**

Vorzugsweise leiten sich die Fettsäure-N-alkylpolyhydroxyalkylamide von reduzierenden Zuckern mit 5 oder 6 Kohlenstoffatomen, insbesondere von der Glucose ab. Die bevorzugten Fettsäure-N-alkylpolyhydroxyalkylamide stellen daher **Fettsäure-N-alkylglucamide** dar, wie sie durch die Formel **(VI)** wiedergegeben werden:

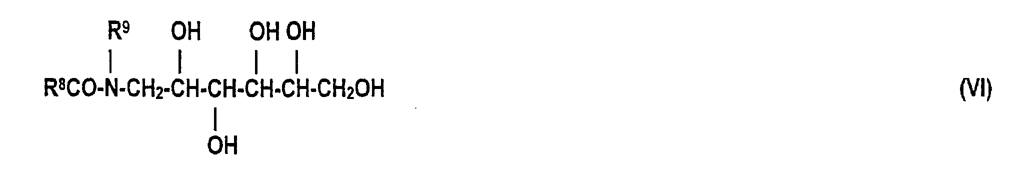

Vorzugsweise werden als Fettsäure-N-alkylpolyhydroxyalkylamide Glucamide der Formel **(VI)** eingesetzt, in der R⁹ für Wasserstoff oder eine Alkylgruppe steht und R⁸CO für den Acylrest der Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Arachinsäure, Gadoleinsäure, Behensäure oder Erucasäure bzw. derer technischer Mischungen steht. Besonders bevorzugt sind Fettsäure-N-alkyl-glucamide der Formel **(VI)**, die durch reduktive Aminierung von Glucose mit Methylamin und anschließende Acylierung mit Laurinsäure oder C_{12/14}-Kokosfettsäure bzw. einem entsprechenden Derivat erhalten werden. Weiterhin können sich die Polyhydroxyalkylamide auch von Maltose und Palatinose ableiten.

### Gewerbliche Anwendbarkeit

Die Siliconersatzstoffe eignen sich zur Herstellung von kosmetischen und/oder pharmazeutischen Zubereitungen, vorzugsweise Haut- und Haarbehandlungsmitteln wie beispielsweise Haarshampoos, Haarlotionen, Schaumbäder, Cremes, Lotionen oder Salben. Sie können femer als weitere Hilfs- und Zusatzstoffe Emulgatoren, Überfettungsmittel, Stabilisatoren, Wachse, Konsistenzgeber, Verdickungsmittel, Kationpolymere, Siliconverbindungen, biogene Wirkstoffe, Antischuppenmittel, Filmbildner, Konservierungsmittel, Hydrotrope, Solubilisatoren, UV-Adsorber, Farb- und Duftstoffe enthalten.

Als **Emulgatoren** kommen beispielsweise nichtionogene Tenside aus mindestens einer der folgenden Gruppen in Frage:
- Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
- C_{12/18}-Fettsäuremono- und -diester von Anlagerungsprodukten von 1 bis 30 Mol Ethylenoxid an Glycerin;
- Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
- Anlagerungsprodukte von 15 bis 60 Mol Ethylenoxid an Ricinusöl und/oder gehärtetes Ricinusöl;
- Anlagerungsprodukte von 2 bis 15 Mol Ethylenoxid an Ricinusöl und/oder gehärtetes Ricinusöl;
- Partialester auf Basis linearer, verzweigter, ungesättigter bzw. gesättigter C_{12/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit) sowie Polyglucoside (z.B. Cellulose);
- Trialkylphosphate;
- Wollwachsalkohole;
- Polysiloxan-Polyalkyl-Polyether-Copolymere bzw. entsprechende Derivate;
- Mischester aus Pentaerythrit, Fettsäuren, Citronensäure und Fettalkohol gemäß **DE-PS 11 65 574** sowie
- Polyalkylenglycole.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE-PS 20 24 051** als Rückfettungsmittel für kosmetische Zubereitungen bekannt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methylquaternierte Difettsäuretriethanolaminester-Salze, besonders bevorzugt sind.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen. Als **Konsistenzgeber** kommen in erster Linie Fettalkohole mit 12 bis 22 und vorzugsweise 16 bis 18 Kohlenstoffatomen in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, kationische Stärke, Copolymere von Diallylammoniumsalzen und Acrylamiden, quaternierte Vinylpyrrolidon/Vinylimidazol-Polymere wie z.B. Luviquat® (BASF AG, Ludwigshafen/ FRG), Kondensationsprodukte von Polyglycolen und Aminen, quaternierte Kollagenpolypeptide wie beispielsweise Lauryldimonium hydroxypropyl hydrolyzed collagen (Lamequat®L, Grünau GmbH), quaternierte Weizenpolypeptide, Polyethylenimin, kationische Siliconpolymere wie z.B. Amidomethicone oder Dow Corning, Dow Corning Co./US, Copolymere der Adipinsäure und Dimethylaminohydroxypropyldiethylentrimamin (Cartaretine®, Sandoz/CH), Polyaminopolyamide wie z.B. beschrieben in der **FR-A 22 52 840** sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen wie z.B. Dibrombutan mit Bisdialkylaminen wie z.B. Bis-Dimethylamino-1,3-propan, kationischer Guar-Gum wie z.B. Jaguar® CBS, Jaguar® C-17, Jaguar® C-16 der Celanese/US, quaternierte Ammoniumsalz-Polymere wie z.B. Mirapol® A-15, Mirapol® AD-1, Mirapol® AZ-1 der Miranol/US.

Geeignete **Siliconverbindungen,** die im Sinne der Erfindung jedoch allenfalls in kleinen Mengen mitverwendet werden sollten, sind beispielsweise Dimethylpolysiloxane, Methylphenylpolysiloxane, cyclische Silicone sowie amino-, fettsäure-, alkohol-, polyether-, epoxy-, fluor- und/oder alkylmodifizierte Siliconverbindungen, die bei Raumtemperatur sowohl flüssig als auch harzförmig vorliegen können. Typische Beispiele für **Fette** sind Glyceride, als **Wachse** kommen u.a. Bienenwachs, Paraffinwachs oder Mikrowachse gegebenenfalls in Kombination mit hydrophilen Wachsen, z.B. Cetylstearylalkohol in Frage. Als **Perlglanzwachse** können insbesondere Mono- und Difettsäureester von Polyalkylenglycolen, Partialglyceride oder Ester von Fettalkoholen mit mehrwertigen Carbonsäuren bzw. Hydroxycarbonsäuren verwendet werden. Als **Stabilisatoren** können Metallsalze von Fettsäuren wie z.B. Magnesium-, Aluminium- und/oder Zinkstearat eingesetzt werden. Unter **biogenen Wirkstoffen** sind beispielsweise Bisabolol, Allantoin, Phytantriol, Panthenol, AHA-Säuren, Pflanzenextrakte und Vitaminkomplexe zu verstehen. Als **Antischuppenmittel** können Climbazol, Octopirox und Zinkpyrethion eingesetzt werden. Gebräuchliche **Filmbildner** sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen. Zur Verbesserung des Fließverhaltens können ferner **Hydrotrope** wie beispielsweise Ethanol, Isopropylalkohol, Propylenglycol oder Glucose eingesetzt werden. Als **Konservierungsmittel** eignen sich beispielsweise Phenoxyethanol, Formaldehydlösung, Parabene, Pentandiol oder Sorbinsäure. Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt- oder Heißprozesse erfolgen; vorzugsweise arbeitet man nach der Phaseninversionstemperatur-Methode.

### Beispiele

Verschiedene Haarshampoos mit den erfindungsgemäßen Siliconersatzstoffen (Rezepturen R1 bis R3+R6) bzw. Silicon (Vergleichsrezepturen R4, R5, R7, R8) wurden von einem Panel von 20 Probanden im bekannten Halbseitentest bezüglich Griff und Glanz auf einer Skala von 1 (= angenehmer Weichgriff, brillanter Glanz) bis 5 (= hart, stumpf) beurteilt. Zur qualitativen Bestimmung der Anreicherung der Ölkörper auf dem Haar wurden Haarsträhnen 10mal abwechselnd mit den Testrezepturen behandelt und getrocknet und anschließend verascht. Eine starke Anreicherung von Ölkörpern wurde in Tabelle mit (+) gekennzeichnet, waren nur geringe oder gar keine Anteile an Ölkörpern nachweisbar, ist dies mit (-) gekennzeichnet. Die Ergebnisse stellen Mittelwerte dar.

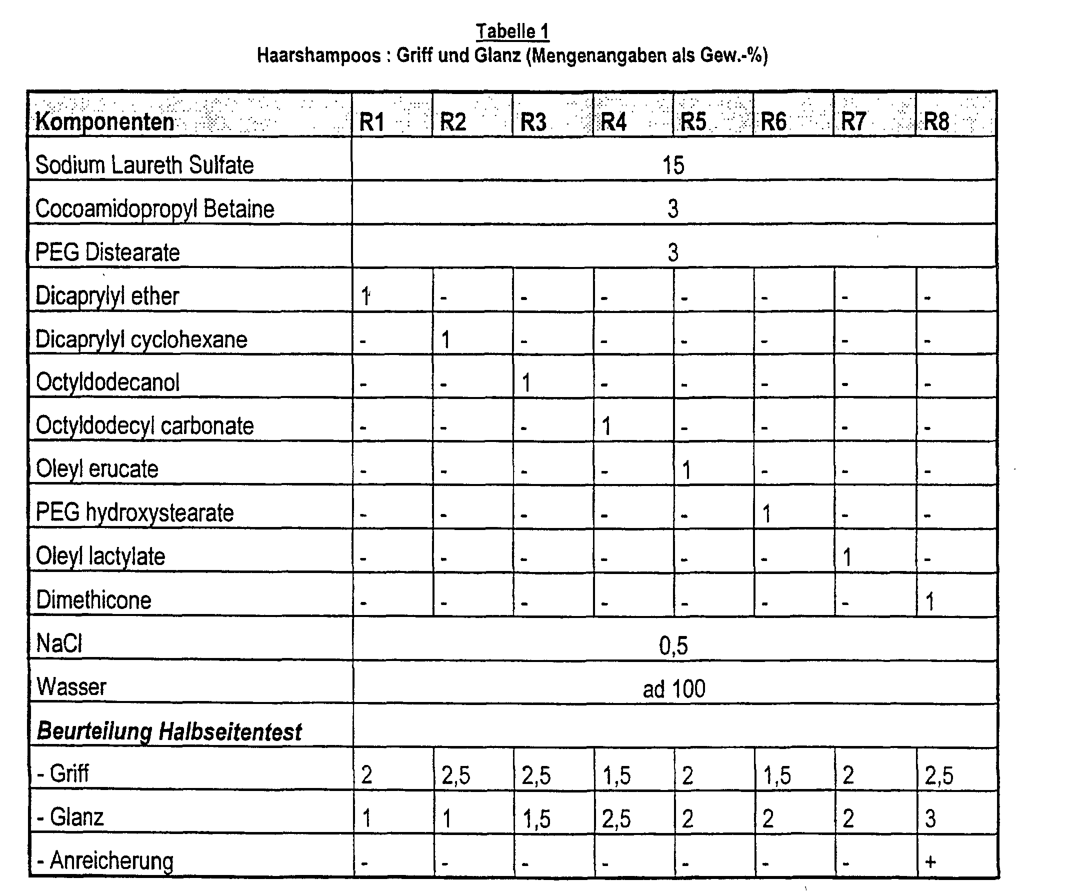

In gleicher Weise wurden die erfindungsgemäßen Haarnachbehandlungsmittel R9 bis R11+R14 sowie die Vergleichsprodukte R12,R13,R15 u. R16 getestet. Die Ergebnisse sind in Tabelle 2 zusammengefaßt.

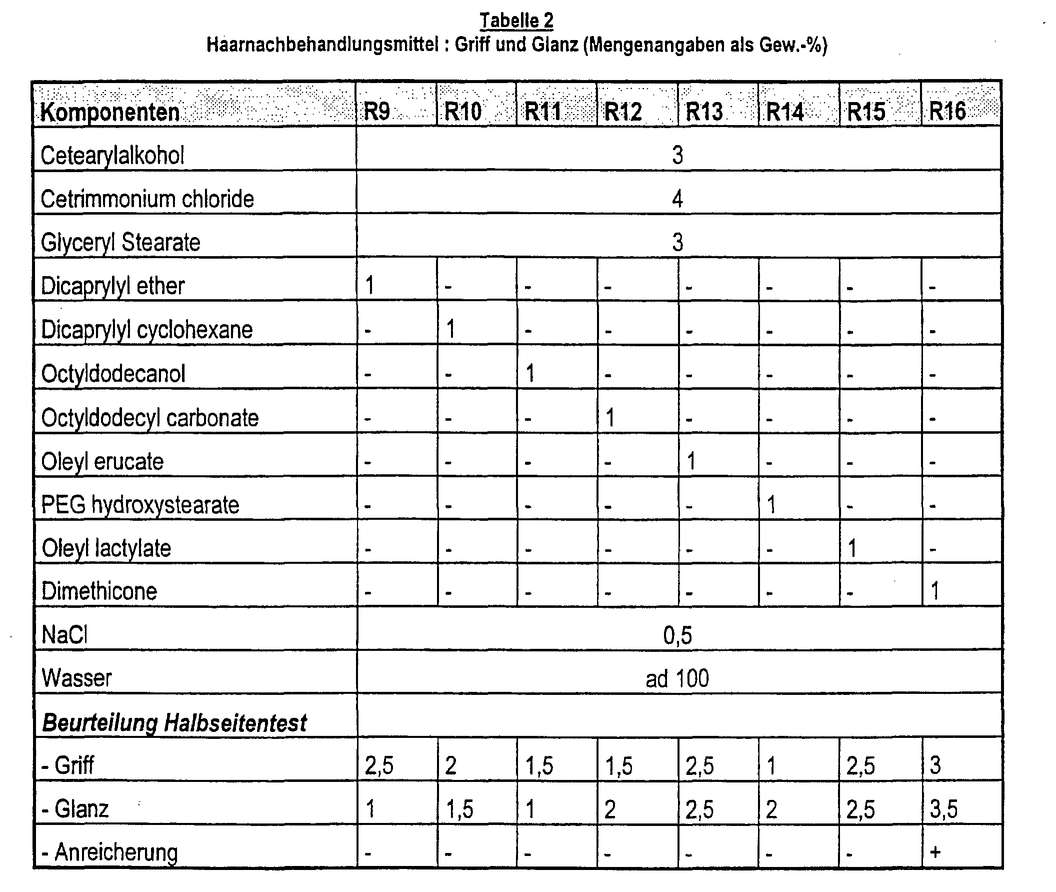

Die Paneltests zeigen, daß unter Verwendung der erfindungsgemäßen Fettstoffe Zubereitungen erhalten werden, die in der Anwendung auf dem Haar besser als die Vergleichsrezepturen mit Siliconen beurteilt werden und gleichzeitig den Vorteil aufweisen, daß es nicht zu einem "build up-Effekt" kommt.

## Patentansprüche

1. Verwendung von Fettstoffen als Siliconersatz zur Herstellung von kosmetischen und/oder pharmazeutischen Zubereitungen, **dadurch gekennzeichnet, daß** man Ölkörper einsetzt, die ausgewählt sind aus der Gruppe, die gebildet wird von
(a) Dialkylethem,
(b) Dialkylcyclohexanen,
(c) Guerbetalkoholen,
(d) Polyolpolyhydroxystearaten und/oder
(e) Hydroxycarbonsäureestem.

2. Verwendung nach Anspruch 1, **dadurch gekennzeichnet, daß** man Dialkylether der Formel **(I)** einsetzt,
**R**^{**1**}**-O-R**^{**2**} **(I)**
in der R¹ und R² unabhängig voneinander für einen linearen oder verzweigten Alkyl- und/oder Alkenylrest mit 6 bis 22 Kohlenstoffatomen steht.

3. Verwendung nach den Ansprüchen 1 und 2, **dadurch gekennzeichnet, daß** man Dialkylcyclohexane der Formel (II) einsetzt,
**R**^{**3**}**-[C]-R**^{**4**} **(II)**
in der R³ und R⁴ unabhängig voneinander für einen linearen oder verzweigten Alkyl- und/oder Alkenylrest mit 6 bis 22 Kohlenstoffatomen und C für einen Cyclohexylrest steht.

4. Verwendung nach den Ansprüchen 1 bis 3, **dadurch gekennzeichnet, daß** man Guerbetalkohole einsetzt, die durch Eigenkondensation von linearen und/oder verzweigten Alkoholen mit 6 bis 22 Kohlenstoffatomen erhalten werden.

5. Verwendung nach den Ansprüchen 1 bis 4, **dadurch gekennzeichnet, daß** man Polyglycerinpolyhydroxystearate einsetzt.

6. Verwendung nach den Ansprüchen 1 bis 5, **dadurch gekennzeichnet, daß** man Ester von Hydroxycarbonsäuren mit 3 bis 18 Kohlenstoffatomen mit aliphatischen Alkoholen mit 1 bis 22 Kohlenstoffatomen einsetzt.

7. Verwendung nach den Ansprüchen 1 bis 6, **dadurch gekennzeichnet, daß** man die Fettstoffe zusammen mit nichtionischen Tensiden vom Typ der Alkyl- und/oder Alkenyloligoglykoside und/ oder Fettsäure-N-alkylglucamide einsetzt.

8. Verwendung nach den Ansprüchen 1 bis 7, **dadurch gekennzeichnet, daß** man die Fettstoffe und die nichtionischen Tenside im Gewichtsverhältnis 10 : 90 bis 90 : 10 einsetzt.

## Claims

1. The use of fatty compounds as a silicone substitute in the production of cosmetic and/or pharmaceutical preparations, **characterized in that** oils selected from the group consisting of
(a) dialkyl ethers,
(b) dialkyl cyclohexanes,
(c) Guerbet alcohols,
(d) polyol polyhydroxystearates and/or
(e) hydroxycarboxylic acid esters
are used.

2. The use claimed in claim 1, **characterized in that** dialkyl ethers corresponding to formula **(I)**:
**R**^{**1**}**-O-R**^{**2**} **(I)**
in which R¹ and R² independently of one another represent a linear or branched alkyl and/or alkenyl radical containing 6 to 22 carbon atoms, are used.

3. The use claimed in claims 1 and 2, **characterized in that** dialkyl cyclohexanes corresponding to formula **(II)**:
**R**^{**3**}**-[C]-R**^{**4**} **(II)**
in which R³ and R⁴ independently of one another represent a linear or branched alkyl and/or alkenyl group containing 6 to 22 carbon atoms and C is a cyclohexyl group,
are used.

4. The use claimed in claims 1 to 3, **characterized in that** Guerbet alcohols obtained by self-condensation of linear and/or branched alcohols containing 6 to 22 carbon atoms are used.

5. The use claimed in claims 1 to 4, **characterized in that** polyglycerol polyhydroxystearates are used.

6. The use claimed in claims 1 to 5, **characterized in that** esters of hydroxycarboxylic acids containing 3 to 18 carbon atoms with aliphatic alcohols containing 1 to 22 carbon atoms are used.

7. The use claimed in claims 1 to 6, **characterized in that** the fatty compounds are used together with nonionic surfactants of the alkyl and/or alkenyl oligoglycoside and/or fatty acid-N-alkyl glucamide type.

8. The use claimed in claims 1 to 7, **characterized in that** the fatty compounds and the nonionic surfactants are used in a ratio by weight of 10:90 to 90:10.

## Revendications

1. Utilisation de matières grasses en tant que substituts pour la production de préparations cosmétiques et/ou pharmaceutiques,
**caractérisée en ce qu'**
on met en oeuvre des composés huileux qui sont choisis dans le groupe formé :
a) des éthers de dialkyle,
b) des dialkylcyclohexanes,
c) des alcools de Guerbet,
d) des stéarates de polyols polyhydroxylés et/ou
e) des esters d'acide hydroxycarboxylique.

2. Utilisation selon la revendication 1,
**caractérisée en ce qu'**
on met en oeuvre des éthers de dialkyle de formule (I)
R¹-O-R² (I)
dans laquelle R¹ et R² indépendamment l'un de l'autre, représentent un reste alkyle et/ou alkényle linéaire ou ramifié ayant de 6 à 22 atomes de carbone.

3. Utilisation selon les revendications 1 et 2,
**caractérisée en ce qu'**
on met en oeuvre des dialkylcyclohexanes de formule (II)
R³-[C]-R⁴ (II)
dans laquelle R³ et R⁴ indépendamment l'un de l'autre, représentent un reste alkyle et/ou alkényle, linéaire ou ramifié, ayant de 6 à 22 atomes de carbone et C représente un reste cyclohexyle.

4. Utilisation selon les revendications 1 à 3,
**caractérisée en ce qu'**
on met en oeuvre des alcools de Guerbet qui sont obtenus par auto-condensation d'alcools linéaires et/ou ramifiés, ayant de 6 à 22 atomes de carbone.

5. Utilisation selon les revendications 1 à 4,
**caractérisée en ce qu'**
on met en oeuvre des polyhydroxystéarates de polyglycérol.

6. Utilisation selon les revendications 1 à 5,
**caractérisée en ce qu'**
on met en oeuvre des esters d'acide hydroxycarboxylique ayant de 3 à 18 atomes de carbone, avec des alcools aliphatiques ayant de 1 à 22 atomes de carbone.

7. Utilisation selon les revendications 1 à 6,
**caractérisée en ce qu'**
on met en oeuvre les matières grasses conjointement avec des agents tensioactifs non ioniques du type des alkyl- et/ou alkényloligoglycosides et/ou des N-alkylglucamides d'acide gras.

8. Utilisation selon les revendications 1 à 7,
**caractérisée en ce qu'**
on met en oeuvre les matières grasses et les agents tensioactifs non ioniques dans un rapport en poids allant de 10 : 90 à 90 : 10.
